# EUROPÄISCHE PATENTSCHRIFT

(11) **EP 3 596 914 B1**
(45) Veröffentlichungstag und Bekanntmachung des Hinweises auf die Patenterteilung: **21.04.2021**
(21) Anmeldenummer: 18714730.1
(22) Anmeldetag: 16.03.2018
(51) Int. Cl.: H04N 1/60, G01J 3/50, G01J 3/52, H04N 17/02, H04N 9/73, H04N 1/401, G01J 3/02

(54) **FARBREFERENZ ZUR KALIBRIERUNG EINER DENTALEN FARBKAMERA**
COLOUR REFERENCE FOR CALIBRATING A DENTAL CAMERA
NUANCIER POUR LA CALIBRATION D'UNE CAMÉRA DENTAIRE

(30) Priorität: 17.03.2017 DE 102017204463
(43) Veröffentlichungstag der Anmeldung: 22.01.2020
(73) Patentinhaber: Sirona Dental Systems GmbH, 64625 Bensheim (DE)
(72) Erfinder: FRANKE, Frederike, 64297 Darmstadt (DE); MEYER, Marcel, 64625 Bensheim (DE); STEGER, Teena, 64646 Heppenheim (DE)
(74) Vertreter: Özer, Alpdeniz
(86) Internationale Anmeldenummer: PCT/EP2018/056671
(87) Internationale Veröffentlichungsnummer: WO 2018/167274

(56) Entgegenhaltungen:
- WO-A1-2008/115547
- DE-A1-102011 050 564
- US-B1- 8 368 762

## Beschreibung

### Technisches Gebiet

Die Erfindung betrifft eine Farbreferenz und ein Verfahren zur Kalibrierung einer dentalen Farbkamera.

### Stand der Technik

Aus dem Stand der Technik sind mehrere Farbreferenzen zur Kalibrierung von Farbkameras bekannt.

Bei einer bekannten Farbreferenz sind auf einer Vorderseite farbige Felder zur Durchführung einer Farbkalibrierung und auf einer Rückseite weiße und graue Felder zur Durchführung eines Weißabgleiches und einer Shading-Korrektur angeordnet.

Die Anzahl der farbigen Felder auf der Vorderseite kann beispielsweise 24 betragen, wobei die Farben der Farbfelder einen bestimmten Spektralbereich abdecken können für den die Farbkalibrierung durchzuführen ist. In einem Schritt wird der Weißabgleich unter Verwendung der Rückseite durchgeführt und in einem zweiten Schritt wird die Farbkalibrierung unter Verwendung der Vorderseite durchgeführt.

WO 2012/038474 A1 offenbart ein Verfahren zum Bestimmen der Farbe von Zähnen mittels einer Farbkarte, die auf einem Lippenspreizer positioniert wird. Die Farbkarte kann dabei bekannte Farbsätze von Standardfarben enthalten, wie RGB oder CMYK.

US 2012/0300228 A1 offenbart eine Farbkarte zur Vorhersage von Druckfarben eines Farbdruckers, wobei die einzelnen Farbfelder in einem Raster aus Quadraten angeordnet sind.

WO 2015/082300 A1 offenbart eine Vorrichtung zur Unterscheidung der Farben von Zähnen mittels eines

Smartphones, umfassend eine Kamera. Mittels eines Abstandshalters wird ein erster Polarisationsfilter, ein zweiter Polarisationsfilter sowie eine Farbtafel in einem festgelegten Abstand relativ zur Kamera des Smartphones angebracht.

EP 0561228 A2 offenbart ein Verfahren und eine Anordnung zur Farbbildendoskopie mit Farbtransformation, wobei während der Aufnahme Farbreferenzen mit aufgenommen werden. Die gemessenen Ist-Farbwerte werden dann mit den Soll-Farbwerten verglichen, um eine Farbkalibrierung durchzuführen.

DE 102011050564 A1 offenbart ein Verfahren zum Farbabgleich beim Drucken digitaler Fotografien, wobei ein ursprüngliches Testbild ausgedruckt und fotografiert wird. Das Testbild weist dabei Felder mit einer Referenzfarbe auf, die über das Bild verteilt sind, wobei aufgrund von Farbabweichungen der Referenzfarbe im gedruckten und fotografierten Bild alle Farben ortsabhängig korrigiert werden.

US 8,368,762 B1 offenbart ein Verfahren und eine Vorrichtung zur Kalibrierung einer Kamera, wobei eine Korrektur von Aberrationen in Bildern insbesondere von chromatischen Aberrationen durchgeführt wird. Dabei schätzt ein mathematisches Modell die chromatischen Aberrationen anhand von Informationen aus einem Kalibrierungsdiagramm.

WO 2008/115547 A1 offenbart ein Verfahren zur automatischen Bildkalibrierung, wobei eine ungleichförmige Beleuchtung korrigiert wird und ein Farbkalibrierungsalgorithmus angewendet wird.

Ein Nachteil der bekannten Farbreferenzen und Verfahren besteht darin, dass die Farbkalibrierung und der Weißabgleich in unterschiedlichen Schritten durchgeführt werden.

Die Aufgabe der vorliegenden Erfindung besteht daher darin, eine vollständige Kalibrierung einer Farbkamera umfassend eine Farbkalibrierung und einen Weißabgleich auf eine möglichst einfache Art und Weise durchzuführen.

### Darstellung der Erfindung

Die Erfindung betrifft eine Farbreferenz gemäß Anspruch 1 zur Kalibrierung einer dentalen Farbkamera umfassend mehrere Felder. Eine Oberfläche der Farbreferenz weist dabei sowohl farbige Felder für eine Farbkalibrierung als auch weiße Felder und/oder graue Felder für einen Weißabgleich und eine Shading-Korrektur auf.

Die Farbreferenz kann beispielsweise eine rechteckige Platte sein, die auf derselben Seite sowohl die farbigen Felder als auch die grauen Felder und/oder weißen Felder aufweist. Durch den Weißabgleich und eine Shading-Korrektur werden auch lokale Änderungen der Helligkeit im Bildfeld, wie die Vignettierung, inhomogene Ausleuchtung oder chromatische Aberrationen, korrigiert. Beim Weißabgleich und der Shading-Korrektur werden also gemessene Ist-Werte der Helligkeit und/oder des Farbtons der weißen Felder über das ganze Bildfeld bestimmt und mit Soll-Werten dieser weißen Felder verglichen. Durch den Weißabgleich und die Shading-Korrektur kann beispielsweise der Abfall der Helligkeit zum Rand des Bildfeldes hin, die sogenannte Vignettierung, korrigiert werden.

Die Shading-Korrektur entspricht einem lokalen Weißabgleich, wobei alle Pixel der optischen Aufnahme unter Verwendung von Kalibrierungsdaten auf denselben Grauwert gesetzt werden.

Zur Durchführung des Weißabgleichs und der Shading-Korrektur können sowohl weiße Felder als auch dunklere graue Felder verwendet werden.

Die farbigen Felder werden für die Farbkalibrierung verwendet, wobei die Farben der farbigen Felder gleichmäßig innerhalb oder auch leicht außerhalb eines zu kalibrierenden Farbspektrums bzw. eines Farbraums angeordnet sein können. Für die einzelnen farbigen Felder werden also Ist-Werte der Helligkeit und/oder der Farbwerte bestimmt und mit bekannten Soll-Werten dieser farbigen Felder verglichen.

Aus dem Vergleich der Ist-Werte mit den Soll-Werten der weißen Felder werden für alle Pixel der optischen Aufnahme Kalibrierungsdaten ermittelt.

Die Kalibrierungsdaten des Weißabgleichs und der Shading-Korrektur können beispielsweise in Form einer Transformationsmatrix abgespeichert werden.

Aus dem Vergleich der Ist-Werte mit den Soll-Werten der farbigen Felder werden die Kalibrierungsdaten der einzelnen Farben ermittelt.

Die Kalibrierungsdaten der Farbkalibrierung der einzelnen Farben können beispielsweise auch in Form einer Lookup-Tabelle abgespeichert werden.

Die farbigen Felder können beispielsweise die folgenden Farben aufweisen: gelb, hellbraun, schwarz, rotbraun, hellgrau, hellblau, hellviolett, hellorange, dunkelgrün, dunkelviolett, dunkelblau, pink, hellgrün, mittelgelb.

Die Soll-Werte für die Farbwerte und die Helligkeitswerte der farbigen Felder und der weißen Felder der jeweiligen Farbreferenz sind vor der Kalibrierung bereits bekannt und können beispielsweise in einem Speicher gespeichert sein.

Ein Vorteil der Farbreferenz liegt darin, dass sowohl der Weißabgleich und die Shading-Korrektur als auch die Farbkalibrierung in einem Schritt durch die Vermessung lediglich einer optischen Aufnahme durchgeführt werden können.

Die Felder weisen eine rechteckige Form auf, wobei die farbigen Felder und die weißen bzw. grauen Felder abwechselnd in Form eines Schachbretts zueinander angeordnet sind.

Durch die schachbrettförmige Anordnung sind die farbigen Felder und die weißen Felder in jeder Richtung abwechselnd zueinander angeordnet. Dies ermöglicht eine gleichmäßige Verteilung der weißen Felder über das gesamte Bildfeld.

Vorteilhafterweise können die farbigen Felder mindestens drei unterschiedliche Farben aufweisen.

Dadurch kann eine grobe Farbkalibrierung durchgeführt werden. Die Anzahl der farbigen Felder kann auch 24 oder mehr betragen. Eine höhere Anzahl der unterschiedlichen farbigen Felder ermöglicht eine genauere Farbkalibrierung.

Die weißen Felder und/oder die grauen Felder sind gleichmäßig auf der Oberfläche der Farbreferenz verteilt.

Durch die gleichmäßige Verteilung der weißen Felder wird ein Weißabgleich und eine Shading-Korrektur über das gesamte Bildfeld ermöglicht.

Vorteilhafterweise können die Felder eine quadratische Form aufweisen.

Durch die quadratische Form der einzelnen Felder wird eine kompakte Ausgestaltung der Farbreferenz ermöglicht.

Vorteilhafterweise können die Felder unmittelbar benachbart sein.

Dadurch wird eine kompakte Ausgestaltung der Farbreferenz ermöglicht. Die Segmentierung der einzelnen Felder wird ebenfalls vereinfacht, da keine Zwischenbereiche vorhanden sind.

Vorteilhafterweise kann die Farbreferenz eine Halterung aufweisen, wobei die Halterung an der Farbkamera anbringbar ist, um die Farbreferenz in einem definierten Abstand und mit einer definierten Ausrichtung relativ zur Farbkamera anzuordnen.

Dadurch kann die Farbreferenz mittels der Halterung relativ zur Farbkamera so angeordnet werden, dass das Bildfeld der Farbkamera die gesamte Farbreferenz abdeckt.

Die Erfindung betrifft weiterhin ein Verfahren gemäß Anspruch 6 zur Kalibrierung einer dentalen Farbkamera unter Verwendung der Farbreferenz. Mittels der dentalen Farbkamera wird die Farbreferenz aufgenommen, wobei eine optische Aufnahme erzeugt wird, wobei für mindestens ein Pixel von mindestens vier der aufgenommenen weißen Felder bzw. grauen Felder ein Farbwert und/oder mindestens ein Helligkeitswert bestimmt wird und der Weißabgleich und die Shading-Korrektur durchgeführt wird, wobei für mindestens drei der aufgenommenen farbigen Felder ein Ist-Farbwert bestimmt wird und mit einem abgespeicherten Soll-Farbwert des jeweiligen farbigen Feldes verglichen wird, wobei Weißabgleich-Kalibrierungswerte für jedes Pixel der optischen Aufnahme zur Durchführung des Weißabgleichs bestimmt werden und Farbkalibrierungswerte für jede Farbe der farbigen Felder bestimmt werden.

Der Farbwert und/oder der Helligkeitswert für ein Pixel kann beispielsweise in Form eines RGB-Wertes verwendet werden.

Im ersten Schritt werden also der Weißabgleich und die Shading-Korrektur unter Verwendung der weißen Felder durchgeführt, wobei im zweiten Schritt die Farbkalibrierung unter Verwendung der farbigen Felder durchgeführt wird.

Beim Weißabgleich und der Shading-Korrektur wird aus der optischen Aufnahme für mindestens ein aufgenommenes Pixel von mindestens vier der aufgenommenen weißen Felder ein Ist-Wert des Farbwertes und/oder des Helligkeitswertes bestimmt und mit dem bekannten Soll-Wert der weißen Felder verglichen. Anschließend wird für mindestens ein Pixel jedes der weißen Felder ein Kalibrierungswert bestimmt. Für die restlichen Pixel können Kalibrierungswerte beispielsweise durch Interpolation bestimmt werden.

Der Ist-Wert kann auch für jedes Pixel der weißen Felder bestimmt werden und mit dem Soll-Wert verglichen werden. Dadurch wird beispielsweise der Verlauf der Helligkeit über das Bildfeld genauer ermittelt.

Der Ist-Wert kann beispielsweise auch nur für jedes vierte Pixel der weißen Felder ermittelt werden.

Anschließend wird die Farbkalibrierung durchgeführt, wobei für mindestens drei der aufgenommenen farbigen Felder ein Ist-Wert des Farbwertes und/oder des Helligkeitswertes, beispielsweise in Form eines RGB-Wertes, bestimmt wird und mit dem bekannten Soll-Wert des jeweiligen farbigen Feldes verglichen wird. Anschließend wird für jede Farbe der einzelnen farbigen Felder ein Farbkalibrierungswert bestimmt. Für die restlichen Farben des zu kalibrierenden Farbraums können Farbkalibrierungswerte beispielsweise durch Interpolation bestimmt werden.

Die Weißabgleich-Kalibrierungswerte und Shading-Korrektur-Kalibrierungswerte der einzelnen Pixel und die Farbkalibrierungswerte der einzelnen Farben können dann als Kalibrierungsdaten abgespeichert werden.

Ein Vorteil des Verfahrens besteht darin, dass der Weißabgleich, die Shading-Korrektur und die Farbkalibrierung in einem Schritt nach der Durchführung einer optischen Aufnahme der Farbreferenz durchgeführt werden können. Der Weißabgleich, die Shading-Korrektur und die Farbkalibrierung können auch mittels einer Recheneinheit, wie eines Computers, Mikrocomputers oder Mikrochips, ohne eine Benutzerinteraktion ausgeführt werden.

Vorteilhafterweise können unter Verwendung eines Segmentierungsalgorithmus die einzelnen Felder der aufgenommenen optischen Aufnahme segmentiert werden.

Der Segmentierungsalgorithmus erkennt die Grenzen der einzelnen Felder und teilt diese in farbige Felder und weiße Felder auf. Der Segmentierungsalgorithmus kann vollautomatisch mittels der Recheneinheit durchgeführt werden.

Der Weißabgleich und die Shading-Korrektur werden über die gesamte Fläche eines Bildfeldes der dentalen Farbkamera durchgeführt, wobei ein Interpolationsalgorithmus verwendet wird, um anhand der Farbwerte und/oder der Helligkeitswerte der weißen bzw. grauen Felder einen Weißabgleich und eine Shading-Korrektur über das gesamte Bildfeld, umfassend auch Bereiche der farbigen Felder, zu ermitteln.

Der Interpolationsalgorithmus ermöglicht beispielsweise den Verlauf der Helligkeitswerte über das gesamte Bildfeld zu bestimmen. Dabei werden die Helligkeitswerte der einzelnen weißen Felder bestimmt, wobei über die farbigen Felder zwischen den weißen Feldern interpoliert wird. Der Interpolationsalgorithmus kann beispielsweise zwischen den einzelnen Helligkeitswerten der weißen Felder eine passende Funktion fitten. Der Interpolationsalgorithmus kann vollautomatisch mittels der Recheneinheit ausgeführt werden.

Vorteilhafterweise kann die Farbreferenz eine Halterung aufweisen, wobei vor der Durchführung der optischen Aufnahme die Farbreferenz mittels der Halterung in einem definierten Abstand und mit einer definierten Ausrichtung relativ zur Farbkamera so angeordnet wird, dass die Abbildung der Farbreferenz in der optischen Aufnahme das gesamte Bildfeld abdeckt.

Durch die Halterung kann die Farbreferenz in einem definierten Abstand mit einer definierten Ausrichtung relativ zur Farbkamera angeordnet werden, so dass das gesamte Bildfeld abgedeckt wird und die Genauigkeit der Kalibrierung verbessert wird.

Vorteilhafterweise kann die Farbreferenz mittels der Halterung senkrecht zu einer Aufnahmerichtung der Farbkamera angeordnet werden.

Dadurch wird die vollautomatische Durchführung der Kalibrierung erleichtert.

Vorteilhafterweise können nach der Durchführung des Weißabgleichs, der Shading-Korrektur und der Farbkalibrierung Kalibrierungsdaten des gesamten Bildfeldes ermittelt werden.

Durch den Vergleich der gemessenen Ist-Werte der Helligkeitswerte und der Farbwerte zu den bekannten Soll-Werten werden die Kalibrierungsdaten über das gesamte Bildfeld ermittelt. Die Kalibrierungsdaten können beispielsweise Korrekturkoeffizienten für die einzelnen Pixel eines Sensors der Farbkamera umfassen.

Vorteilhafterweise können die ermittelten Kalibrierungsdaten in einem Speicher abgespeichert werden, um zur Korrektur auf die optischen Aufnahmen der Farbkamera angewendet zu werden.

Die Kalibrierungsdaten werden also auf die optischen Aufnahmen nach der Durchführung der Kalibrierung angewendet, um eine Korrektur durchzuführen. Die Kalibrierungsdaten können beispielsweise in Form einer Transformationsmatrix oder einer Lookup-Tabelle abgespeichert werden.

### Kurzbeschreibung der Zeichnungen

Die Erfindung wird anhand der Zeichnungen erläutert. Es zeigt, die
- Fig. 1: eine Farbreferenz in Form einer Platte zur Kalibrierung einer dentalen Farbkamera, die
- Fig. 2: eine Farbreferenz mit quadratischen Feldern, die
- Fig. 3: eine Farbreferenz mit rechteckigen Feldern, die
- Fig. 4: eine Farbreferenz mit rautenförmigen Feldern.

### Ausführungsbeispiele

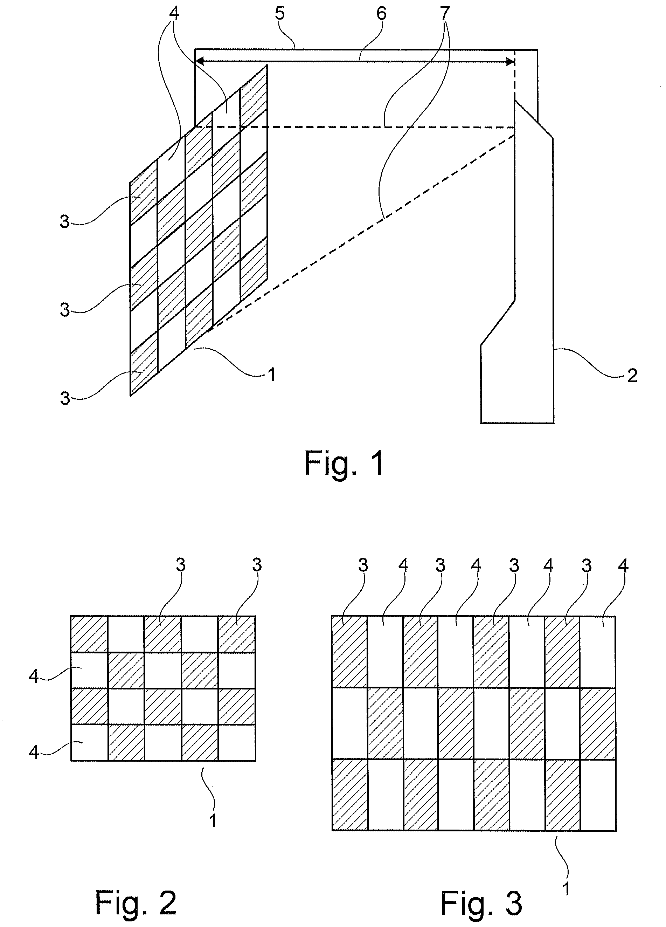

Die Fig. 1 zeigt eine Farbreferenz 1 in Form einer Platte zur Kalibrierung einer dentalen Farbkamera 2, umfassend farbige Felder 3 und weiße Felder 4. In der dargestellten Ausführungsform sind die Felder 3 und 4 quadratisch geformt und schachbrettförmig zueinander angeordnet. An der Farbreferenz 1 ist eine Halterung 5 angeordnet, die an der Farbkamera 2 so anbringbar ist, dass die Farbreferenz 1 in einem definierten Abstand 6 und mit einer festgelegten Ausrichtung relativ zur Farbkamera 2 angeordnet werden kann. Die Farbreferenz 1 wird so angeordnet, dass die Felder 3 und 4 der Farbreferenz 1 das gesamte Bildfeld 7 der Farbkamera 2 abdecken, wobei die Grenzen des Bildfeldes 7 durch die gestrichelten Linien angedeutet sind.

Die Fig. 2 weist eine weitere Ausführungsform der Farbreferenz 1 auf, wobei die farbigen Felder 3 und die weißen Felder 4 schachbrettförmig in vier Zeilen und fünf Reihen angeordnet sind, wobei die Felder 3 und 4 quadratisch geformt sind.

Die Fig. 3 zeigt eine weitere Ausführungsform der Farbreferenz 1, wobei die farbigen Felder 3 und die weißen Felder 4 als längliche Rechtecke geformt sind und schachbrettförmig in drei Zeilen und acht Reihen angeordnet sind.

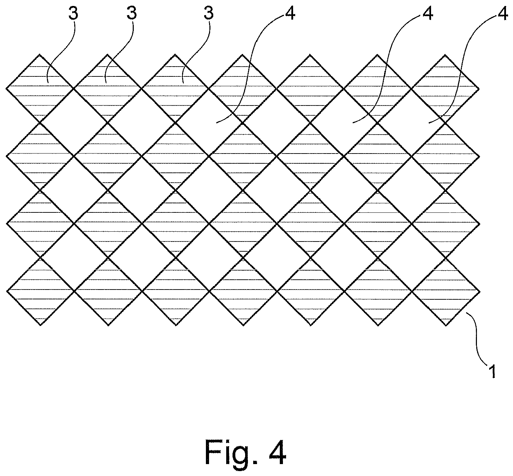

Die Fig. 4 zeigt eine weitere Ausführungsform der Farbreferenz 1, wobei die farbigen Felder 3, die schraffiert dargestellt sind, und die weißen Felder 4 rautenförmig geformt sind und schachbrettförmig angeordnet sind.

Die vorliegende Erfindung stellt bereit ein Verfahren zur Kalibrierung einer dentalen Farbkamera 2 unter Verwendung der Farbreferenz 1,wobei mittels der dentalen Farbkamera 2 die Farbreferenz 1 aufgenommen wird, wobei eine optische Aufnahme erzeugt wird, wobei für mindestens ein Pixel von mindestens vier der aufgenommenen weißen Felder 4 und/oder grauen Felder 4 ein Farbwert und/oder mindestens ein Helligkeitswert bestimmt wird und der Weißabgleich und die Shading-Korrektur durchgeführt wird, wobei für mindestens drei der aufgenommenen farbigen Felder (3) ein Ist-Farbwert bestimmt wird und mit einem abgespeicherten Soll-Farbwert des jeweiligen farbigen Feldes (3) verglichen wird, wobei Weißabgleich-Kalibrierungswerte und Shading-Kalibrierungswerte für Pixel der optischen Aufnahme zur Durchführung des Weißabgleichs und der Shading-Korrektur bestimmt werden und Farbkalibrierungswerte für jede Farbe der farbigen Felder bestimmt werden, wobei der Weißabgleich und die Shading-Korrektur über die gesamte Fläche eines Bildfeldes (7) der dentalen Farbkamera (2) durchgeführt wird, wobei ein Interpolationsalgorithmus verwendet wird, um anhand der Farbwerte und/oder der Helligkeitswerte der weißen Felder (4) und/oder der grauen Felder (4) einen Weißabgleich und eine Shading-Korrektur über das gesamte Bildfeld (7) umfassend auch Bereiche der farbigen Felder (3) zu ermitteln.

### Bezugszeichen

- 1: Farbreferenz
- 2: Farbkamera
- 3: farbige Felder
- 4: weiße Felder
- 5: Halterung
- 6: Abstand
- 7: Bildfeld

## Patentansprüche

1. Farbreferenz (1) zur Kalibrierung einer dentalen Farbkamera (2) umfassend mehrere Felder (3, 4), **dadurch gekennzeichnet, dass** eine Oberfläche der Farbreferenz (1) sowohl farbige Felder (3) für eine Farbkalibrierung als auch weiße Felder (4) und/oder graue Felder (4) für einen Weißabgleich und eine Shading-Korrektur aufweist, wobei die weißen Felder (4) und/oder die grauen Felder (4) gleichmäßig auf der Oberfläche der Farbreferenz (1) verteilt sind, wobei die Felder (3, 4) eine rechteckige Form aufweisen, wobei die farbigen Felder (3) und die weißen Felder (4) und/oder die grauen Felder (4) abwechselnd in Form eines Schachbretts zueinander angeordnet sind.

2. Farbreferenz (1) nach Anspruch 1, **dadurch gekennzeichnet, dass** die farbigen Felder (3) mindestens drei unterschiedliche Farben aufweisen.

3. Farbreferenz (1) nach Anspruch 1 oder 2, **dadurch gekennzeichnet, dass** die Felder (3, 4) eine quadratische Form aufweisen.

4. Farbreferenz (1) nach einem der Ansprüche 1-3, **dadurch gekennzeichnet, dass** die Felder (3, 4) unmittelbar benachbart sind.

5. Farbreferenz (1) nach einem der Ansprüche 1-4, **dadurch gekennzeichnet, dass** die Farbreferenz (1) eine Halterung (5) aufweist, wobei die Halterung (5) an der Farbkamera (2) anbringbar ist, um die Farbreferenz (1) in einem definierten Abstand (6) und mit einer definierten Ausrichtung relativ zur Farbkamera (2) anzuordnen.

6. Verfahren zur Kalibrierung einer dentalen Farbkamera (2) unter Verwendung der Farbreferenz (1) nach einem der Ansprüche 1-5, **dadurch gekennzeichnet, dass** mittels der dentalen Farbkamera (2) die Farbreferenz (1) aufgenommen wird, wobei eine optische Aufnahme erzeugt wird, wobei für mindestens ein Pixel von mindestens vier der aufgenommenen weißen Felder (4) und/oder grauen Felder (4) ein Farbwert und/oder mindestens ein Helligkeitswert bestimmt wird und der Weißabgleich und die Shading-Korrektur durchgeführt wird, wobei für mindestens drei der aufgenommenen farbigen Felder (3) ein Ist-Farbwert bestimmt wird und mit einem abgespeicherten Soll-Farbwert des jeweiligen farbigen Feldes (3) verglichen wird, wobei Weißabgleich-Kalibrierungswerte und Shading-Kalibrierungswerte für Pixel der optischen Aufnahme zur Durchführung des Weißabgleichs und der Shading-Korrektur bestimmt werden und Farbkalibrierungswerte für jede Farbe der farbigen Felder bestimmt werden, wobei der Weißabgleich und die Shading-Korrektur über die gesamte Fläche eines Bildfeldes (7) der dentalen Farbkamera (2) durchgeführt wird, wobei ein Interpolationsalgorithmus verwendet wird, um anhand der Farbwerte und/oder der Helligkeitswerte der weißen Felder (4) und/oder der grauen Felder (4) einen Weißabgleich und eine Shading-Korrektur über das gesamte Bildfeld (7) umfassend auch Bereiche der farbigen Felder (3) zu ermitteln.

7. Verfahren nach Anspruch 6, **dadurch gekennzeichnet, dass** unter Verwendung eines Segmentierungsalgorithmus die einzelnen Felder (3, 4) der aufgenommenen optischen Aufnahme segmentiert werden.

8. Verfahren nach Anspruch 6 oder 7, **dadurch gekennzeichnet, dass** die Farbreferenz (1) eine Halterung (5) aufweist, wobei vor der Durchführung der optischen Aufnahme die Farbreferenz (1) mittels der Halterung (5) in einem definierten Abstand (6) und mit einer definierten Ausrichtung relativ zur Farbkamera (2) so angeordnet wird, dass die Abbildung der Farbreferenz (1) in der optischen Aufnahme das gesamte Bildfeld (7) abdeckt.

9. Verfahren nach Anspruch 8, **dadurch gekennzeichnet, dass** die Farbreferenz (1) mittels der Halterung (5) senkrecht zu einer Aufnahmerichtung der Farbkamera (2) angeordnet wird.

10. Verfahren nach einem der Ansprüche 6-9, **dadurch gekennzeichnet, dass** nach der Durchführung des Weißabgleichs, der Shading-Korrektur und der Farbkalibrierung Kalibrierungsdaten des gesamten Bildfeldes (7) ermittelt werden.

11. Verfahren nach Anspruch 10, **dadurch gekennzeichnet, dass** die ermittelten Kalibrierungsdaten in einem Speicher abgespeichert werden, um zur Korrektur auf die optischen Aufnahmen der Farbkamera (2) angewendet zu werden.

## Claims

1. A colour reference (1) for calibrating a dental camera (2) comprising a plurality of fields (3, 4), **characterised in that** a surface of the colour reference (1) has both coloured fields (3) for colour calibration and white fields (4) and/or grey fields (4) for a white balance and a shading correction, wherein the white fields (4) and/or the grey fields (4) are evenly distributed over the surface of the colour reference (1), wherein the fields (3, 4) have a rectangular shape, wherein the coloured fields (3) and the white fields (4) and/or the grey fields (4) are arranged alternately in the form of a chessboard.

2. The colour reference (1) according to claim 1, **characterised in that** the coloured fields (3) have at least three different colours.

3. The colour reference (1) according to claim 1 or 2, **characterised in that** the fields (3, 4) have a square shape.

4. The colour reference (1) according to any one of claims 1-3, **characterised in that** the fields (3, 4) are directly adjacent to one another.

5. The colour reference (1) according to any one of claims 1-4, **characterised in that** the colour reference (1) has a bracket (5), wherein the bracket (5) is attachable to the camera (2) in order to display the colour reference (1) at a defined distance (6) and with a defined alignment relative to the camera (2).

6. A method for calibrating a dental camera (2) using the colour reference (1) according to any one of claims 1-5, **characterised in that** the colour reference (1) is recorded by means of the dental camera (2), wherein an optical image is generated, wherein a colour value and/or at least one brightness value is determined for at least one pixel of at least four of the recorded white fields (4) and/or grey fields (4) and the white balance and the shading correction are carried out, wherein an actual colour value is determined for at least three of the recorded coloured fields (3) and compared with a stored target colour value of the respective coloured field (3), wherein white balance calibration values and shading calibration values are determined for pixels of the optical image for implementing the white balance and the shading correction and colour calibration values are determined for each colour of the coloured fields, wherein the white balance and the shading correction are implemented over the entire area of an image field (7) of the dental camera (2), wherein an interpolation algorithm is used to determine a white balance and a shading correction over the entire image field (7) also comprising areas of the coloured fields (3) on the basis of the colour values and/or the brightness values of the white fields (4) and/or the grey fields (4).

7. The method according to claim 6, **characterised in that** the individual fields (3, 4) of the recorded optical image are segmented using a segmentation algorithm.

8. The method according to claim 6 or 7, **characterised in that** the colour reference (1) has a bracket (5), wherein, prior to the optical image being recorded, the colour reference (1) is, by means of the bracket (5), arranged at a defined distance (6) and with a defined alignment relative to the camera (2) in such a way that the image of the colour reference (1) in the optical image covers the entire image field (7).

9. The method according to claim 8, **characterised in that** the colour reference (1) is, by means of the bracket (5), arranged perpendicularly to a recording direction of the camera (2).

10. The method according to any one of claims 6-9, **characterised in that** calibration data of the entire image field (7) is determined after the white balance, the shading correction and the colour calibration have been implemented.

11. The method according to claim 10, **characterised in that** the determined calibration data is stored in a memory in order to be used for correction on the optical images of the camera (2).

## Revendications

1. Nuancier (1) pour la calibration d'une caméra dentaire (2) comprenant plusieurs champs (3, 4), **caractérisé en ce qu'**une surface du nuancier (1) comporte à la fois des champs colorés (3) pour une calibration des couleurs et des champs blancs (4) et/ou des champs gris (4) pour un équilibrage des blancs et une correction de l'ombrage, les champs blancs (4) et/ou les champs gris (4) étant uniformément répartis sur la surface du nuancier (1), les champs (3, 4) étant de forme rectangulaire, les champs colorés (3) et les champs blancs (4) et/ou les champs gris (4) étant disposés en alternance sous la forme d'un échiquier.

2. Nuancier (1) selon la revendication 1, **caractérisé en ce que** les champs colorés (3) présentent au moins trois couleurs différentes.

3. Nuancier (1) selon la revendication 1 ou 2, **caractérisé en ce que** les champs (3, 4) sont de forme carrée.

4. Nuancier (1) selon l'une quelconque des revendications 1 à 3, **caractérisé en ce que** les champs (3, 4) sont immédiatement adjacents.

5. Nuancier (1) selon l'une quelconque des revendications 1 à 4, **caractérisé en ce que** ledit nuancier (1) comporte un support (5), le support (5) pouvant être fixé à la caméra (2) pour disposer le nuancier (1) à une distance définie (6) et avec une orientation définie par rapport à la caméra (2).

6. Procédé de calibration d'une caméra dentaire (2) à l'aide du nuancier (1) selon l'une quelconque des revendications 1 à 5, **caractérisé en ce que** le nuancier (1) est enregistré au moyen de la caméra dentaire (2), un enregistrement optique étant généré, dans lequel, pour au moins un pixel d'au moins quatre des champs blancs (4) et/ou des champs gris (4) enregistrés, une valeur de couleur et/ou au moins une valeur de luminosité est/sont déterminée(s) et l'équilibrage des blancs et la correction de l'ombrage sont effectués, dans lequel pour au moins trois des champs colorés enregistrés (3), une valeur de couleur réelle est déterminée et comparée à une valeur de couleur cible mémorisée du champ coloré (3) correspondant, les valeurs de calibration de l'équilibrage des blancs et les valeurs de calibration de l'ombrage étant déterminées pour les pixels de l'enregistrement optique afin d'effectuer l'équilibrage des blancs et la correction de l'ombrage, et les valeurs de calibration des couleurs étant déterminées pour chaque couleur des champs colorés, l'équilibrage des blancs et la correction de l'ombrage étant effectués sur toute la surface d'un champ d'image (7) de la caméra dentaire (2), un algorithme d'interpolation étant utilisé pour obtenir un équilibrage des blancs et une correction d'ombrage sur tout le champ d'image (7), comprenant également des zones des champs colorés (3), en se fondant sur les valeurs de couleur et/ou les valeurs de luminosité des champs blancs (4) et/ou des champs gris (4).

7. Procédé selon la revendication 6, **caractérisé en ce que** les champs individuels (3, 4) de l'image optique enregistrée sont segmentés à l'aide d'un algorithme de segmentation.

8. Procédé selon la revendication 6 ou 7, **caractérisé en ce que** le nuancier (1) comporte un support (5), le nuancier (1) étant disposé, au moyen du support (5), à une distance définie (6) et avec une orientation définie par rapport à la caméra (2), avant que l'enregistrement optique ne soit effectué, de telle sorte que l'image du nuancier (1) couvre dans l'enregistrement optique tout le champ d'image (7).

9. Procédé selon la revendication 8, **caractérisé en ce que** le nuancier (1) est disposé, au moyen du support (5), perpendiculairement à une direction d'enregistrement de la caméra (2).

10. Procédé selon l'une quelconque des revendications 6 à 9, **caractérisé en ce qu'**après la mise en oeuvre de l'équilibrage des blancs, de la correction de l'ombrage et de la calibration des couleurs, des données de calibration de l'ensemble du champ d'image (7) sont obtenues.

11. Procédé selon la revendication 10, **caractérisé en ce que** les données d'de calibration obtenues sont mémorisées dans une mémoire pour être utilisées pour corriger des enregistrements optiques de la caméra (2).
